# EUROPEAN PATENT APPLICATION

(11) **EP 4 300 505 A1**
(43) Date of publication of application: **03.01.2024**
(21) Application number: 22181784.4
(22) Date of filing: 29.06.2022
(51) Int. Cl.: G16H 10/60, G16H 50/20

(54) **MEDICAL STRUCTURED REPORTING WORKFLOW ASSISTED BY NATURAL LANGUAGE PROCESSING TECHNIQUES**

(71) Applicant: Ebit srl, 16152 Genova (IT)
(72) Inventor: Ferrando, Giovanni, 16156 Genova Pegli (IT); Minetti, Irene, 15074 Molare (AL) (IT)
(74) Representative: Bessi, Lorenzo

(57) **Abstract**

A computer-implemented method for managing a medical structured reporting workflow comprising:
a) receiving a structured reporting workflow designator designating an individual structured report template from a class of structured report templates, such structured report templates having fields to be populated with data received in reply to a specific question;
b) receiving a free text medical report related to the designated structured report template;
c) processing the free text medical report with a machine learning algorithm to determine answers to the questions associated to the fields of the designated structured report template;
d) using the answers to populate the fields of the designated structured report template with associated data.

A corresponding system and computer program are also disclosed.

## Description

### FIELD OF THE INVENTION

The present disclosure relates to the technical field of medical reporting and more particularly to structured medical reporting workflows inside diagnostic imaging departments.

### STATE OF THE ART

In diagnostic imaging departments, various equipment is provided to afford image guidance in connection with diagnosis and treatment of disease. A broad range of examinations and procedures may be performed. Non-limiting examples of fields of applications are radiology and cardiology procedures, both interventional or diagnostic.

A wide range of imaging modalities may be used to afford image guidance, such as x-ray equipment, CT equipment, ultrasound, MRI and other imaging modalities.

Starting from the preparation of the patient until completion of a medical procedure, a physician, technician or other medical personnel record various information regarding the patient and procedure into one or more reports to be maintained, such as with the patient's records. Various information systems are used today in connection with generating patient records and reports. For example, radiology information systems (RIS) or cardiovascular information systems (CVIS) are utilized in numerous imaging departments and elsewhere within hospitals and complement hospital information systems (HIS) and picture archiving and communications systems (PACS) in an effort to afford efficient workflow for medical personnel. The various functions afforded by such systems, also called Enterprise Imaging Reporting System, may include patient scheduling, resource management, examination performance tracking, examination interpretation, results distribution and procedure billing. An example of a commercial Enterprise Imaging Reporting System is offered by Ebit S.r.l. of Genoa, Italy, under the tradename SUITESTENSA. The SUITESTENSA system is an imaging and information management software platform that is described more fully at: http://www.esaote.com/en-US/healthcare-it/healthcare-it-software/p/suitestensacvis-pacs/ and at: http://www.esaote.com/en-US/healthcare-it/healthcare-it-software/p/suitestensa-ris-pacs/.

However, conventional information systems still experience certain limitations. As one example, no specific data collection tool or information system is provided to facilitate and automate structured data collection phase.

Current approaches to structured reporting often require a predetermined configuration of several templates to be manually filled in by a radiologist/cardiologist. Such activity is time consuming and often refused by physicians, that prefer dictating or writing free text.

The large amount of unstructured information in full-text reports is a valuable source of information to increase the quality of physicians' work and more generally of clinical care and the creation of scientific databases. The penetration of these applications is much less widespread depending on the difficulties deriving from the analysis of "narrative" and linguistic data.

At the same time, organizing the medical report using standard schemes and phrases makes it more complete, easily comparable with previous and subsequent ones, unmistakable for doctors and patients who must use the information contained in it correctly.

"Structuring" the report doesn't mean simply being able to standardize it in the vocabulary and format but generating it in such a way that the therein encoded information allows a "semantic" understanding of the data that is essential for the subsequent storage within an Electronic Health Record (EHR) or a Vendor Neutral Archive (VNA).

In the most currently used HIS systems there is already the possibility to organize the reports either with the help of pre-defined formats or with numerous standard reports (negative chest report, BPCO chest, etc.) or with the use of predefined phrases chosen by the user.

The structured report, however, is a computer document divided into significant, orderly and coherent sections using a standardized language. In this sense, a report, in order to be structured, must be based on schematics (template), measurements and checklists that depend on the survey carried out, the imaging modality and the district. It may also contain supplementary information collected during the examination and useful for diagnosis, such as, for example, clinical and anamnestic data, technical parameters, measurements, annotations and multimedia content.

On the other hand, the constant increase in the volume of request for radiological services forces the radiologist to constantly have to mediate the timing available for his report with other factors such as the medical time necessary to be able to fully draft it, the daily workload, the moments dedicated to the necessary dialogue with the patient and with colleagues, the acquisition of informed consent, and more generally with the need to organize the activity of the diagnostic room as a whole.

The inventors made several investigations to verify if artificial intelligence could help in the solution to the problem of smart structured medical reporting.

Despite the recent successes of machine learning and deep learning techniques applied to language processing, these methods, however, often require several thousand examples in order to effectively model the information present in complex documents. See, for example Ferraro et al. "Improving performance of natural language processing part-of-speech tagging on clinical narratives through domain adaptation", Journal of the American Medical Informatics Association, Volume 20, Issue 5, September 2013, Pages 931-939. This makes them particularly difficult to apply in the clinical field, where data available for research purposes are difficult to find.

Therefore, a need remains for improved systems and methods that address the above noted difficulties, as well as other problems that will become available upon reading the text herein.

### SUMMARY OF THE INVENTION

It is thus an object of embodiments herein to provide methods, computer program products and systems that facilitate collection of patient data, clinical data, procedural data as well as information about the materials and therapies, in a structured manner.

A further object is to provide methods, computer program products and systems that facilitate generation of structured reports that are easy to read and include aspects that are automatically derived, also providing checking inconsistency and alerts.

A further object is to provide structured reports that record all data of interest in a manner that is available for statistical and clinical research, as well as for extraction to national registries and the like.

In accordance with embodiments herein, systems, computer program products and computer implemented methods are provided for managing medical structured reporting, the systems, program products and methods comprising, under control of one or more computer systems configured with specific executable instructions:
- receiving a structured reporting workflow designator designating an individual structured report template from a class of structured report templates, optionally automatically selected from the available templates according to exam type or clinical question, such structured report templates having fields to be populated with data received in reply to a specific question, for example derived by clinical measurements and/or by selecting from a list of predefined items, wherein each item of each list is associated to a specific answer to a specific question;
- receiving a free text - written or dictated - medical report related to the designated structured report template;
- processing the free text medical report with a machine learning algorithm of the question answering type to determine answers to the questions associated to the fields of the designated structured report template;
- using the answers to populate the fields of the designated structured report template with associated data.

To reduce time, optimize the workflow and avoid errors, the inventors decided to focus on the implementation of natural language processing (NLP) techniques in order to catalog anamnestic and/or diagnostic and/or therapeutic data and create a scientific database to compare such data with similar cases and/or guidelines to provide additional information that can facilitate the doctor during the reporting process and to guide in real time the presentation of medical images in terms of display protocols, available tools and study comparison.

Using an NLP approach to build a structured report would allow to:
- provide medical staff with a dashboard in which the salient events that distinguish the patient's clinical history are summarized, with the possibility of loading the most relevant previous imaging studies and applying a whole series of automatisms that allow an optimized analysis of the study in question;
- support the doctor in the patient management to predict the outcome of the procedure and its follow-up over time through a comparison with the studies present in a scientific database;
- reduce the clinical risk resulting from an inconsistent drafting of the report through semantic validation rules, checks relating to the mandatory nature of certain data or consistency between entered data and DICOM tags associated with imaging studies.
- Easily classify studies both for scientific purposes and to improve the training of NLP systems through the extraction of TAGs.
- Identify worklist prioritization criteria, to report the most urgent patients automatically and promptly by changing the priority level of an examination within the reporting list.

Some NLP (Natural Language Processing) approaches for structuring information from dictated text are available in the state of the art (see, for example, Spandorfer et Al, "Deep learning to convert unstructured CT pulmonary angiography reports into structured reports", Eur Radiol Exp 3, 37, 2019), but their goal is more focused to divide and label the sections of the report , rather than automatically fill in and check the clinical information into specific predefined fields. Alternatively, NLP algorithms, combined with more standard feature extraction and pattern recognition techniques, are used to retrieve from a clinical archive and display to the user previous examinations and reports based on a similarity score.

Another application in clinical support services include using NLP in assisting radiologists at the time of interpretation, including providing clinical decision support for the radiologists on various pathologies and on clinical risk by detecting errors within radiology reports related for example to laterality and sex.

Moreover, in such systems there is no evidence of effects on the image workstation, in terms of presentation state, hanging protocols and available tools.

In order to fully exploit the capabilities of modern natural language processing systems without compromising the effectiveness and generalization of the approach, in an embodiment, a question answering model has been adopted.

Such model is, for example, the one called UnifiedQA as disclosed in Khashabi et al. 2020, "UNIFIEDQA: Crossing Format Boundaries with a Single QA System", Findings of the Association for Computational Linguistics: EMNLP 2020, which is based on a neural network pre-trained in various formats (extractive, abstractive, multiple choice and true/false). In particular, the generalization of the chosen model by not requiring any type of domain specific training can therefore be easily extended to other types of reports without requiring a dedicated training procedure for each.

In an embodiment, the machine learning algorithm is of the question-answering type pre-trained on known datasets of questions-answers including one or more of the following formats: Extractive where questions include a context paragraph, typically a paragraph, and require models to extract the answer as a substring from the context, Abstractive where questions require models to produce answers that are often not mere substrings of the provided context paragraph, Multiple-choice where questions have a set of candidate answers of which generally exactly one is correct, Yes/No where questions expect a 'yes' or 'no' answer as the response.

The populated fields of the designated structured report template may be advantageously subjected to a review for being corrected and, possibly, used for retraining the machine learning algorithm thus improving its reliability.

Further improvements are the object of the sub claims.

### BRIEF DESCRIPTION OF THE DRAWINGS

The characteristics of the invention and the advantages derived therefrom will be more apparent from the following description of non-limiting embodiments, illustrated in the annexed drawings, in which:
FIG. 1 illustrates a block diagram of an interventional radiology department formed in accordance with embodiments herein.
FIG. 2 illustrates an example block diagram of an interventional radiology information system formed in accordance with embodiments herein.
FIG. 3 illustrates an example of at least a portion of the resources utilized in an interventional radiology structured workflow.
FIG. 4 illustrates an example of a query applied on a context to determine a field of attributes.
FIG. 5 illustrates an example on how the Field "Tosse" in Italian (cough) is automatically populated by answering Yes or No to the question "Do patient symptoms include cough" processing the text "Patient's symptoms include fever and diarrhea, without coughing" translated from the Italian text "II paziente presenta sintomi quali febbre e diarrea, in assenza di tosse".
FIG. 6 illustrates another example of a free-text report in Italian which is automatically translated into English and then processed by a UnifiedQA algorithm by formulating a specific question.
FIG. 7 illustrates a process for processing a medical report involving also a step of expert review to be used for retraining the UnifiedQA algorithm.

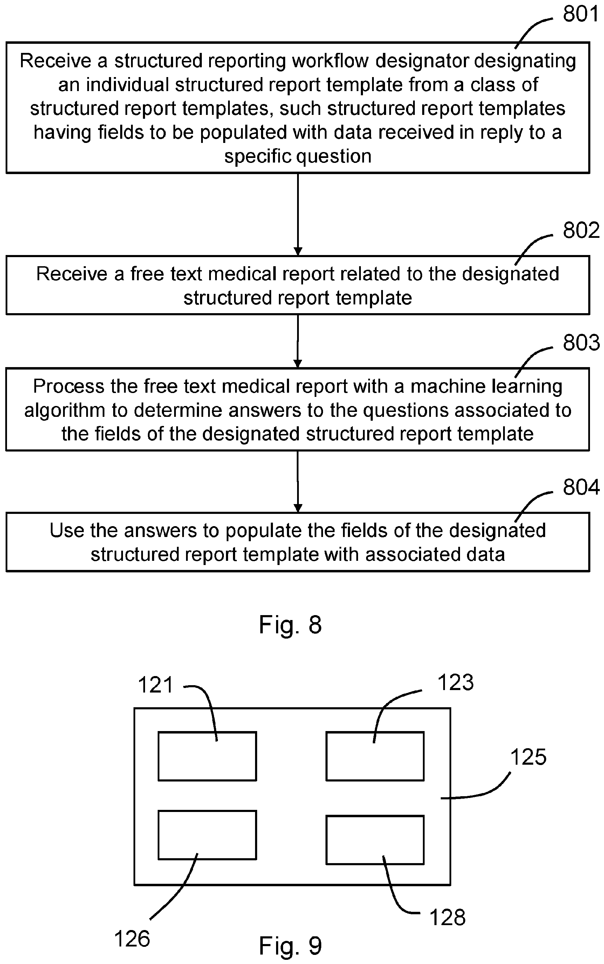

FIG. 8 illustrates a process carried out in accordance with embodiments herein for generating structured reports.
FIG. 9 illustrates an exemplary workstation for carrying out the process of FIG. 8.

### DETAILED DESCRIPTION OF A PREFERRED EMBODIMENT

The embodiments of the systems and methods described herein may be implemented in hardware or software, or a combination of both. However, preferably, these embodiments are implemented in computer programs executing on programmable computers each comprising at least one processor, a data storage system (including volatile and non-volatile memory and/or storage elements), at least one input device, and at least one output device. For example and without limitation, the programmable computers may be a personal computer, laptop, personal data assistant, and cellular telephone. Program code is applied to input data to perform the functions described herein and generate output information. The output information is applied to one or more output devices, in known fashion.

Each program is preferably implemented in a high level procedural or object oriented programming and/or scripting language to communicate with a computer system. However, the programs can be implemented in assembly or machine language, if desired. In any case, the language may be a compiled or interpreted language. Each such computer program is preferably stored on a storage media or a device readable by a general or special purpose programmable computer, for configuring and operating the computer when the storage media or device is read by the computer to perform the procedures described herein. The inventive system may also be considered to be implemented as a computer-readable storage medium, configured with a computer program, where the storage medium so configured causes a computer to operate in a specific and predefined manner to perform the functions described herein.

Structured reporting is a requirement involving all departments of a medical environment, particularly the diagnostic imaging departments. By way of a non-limiting example, in the following pages the workflow leading to a structured report in an interventional radiology department is described.

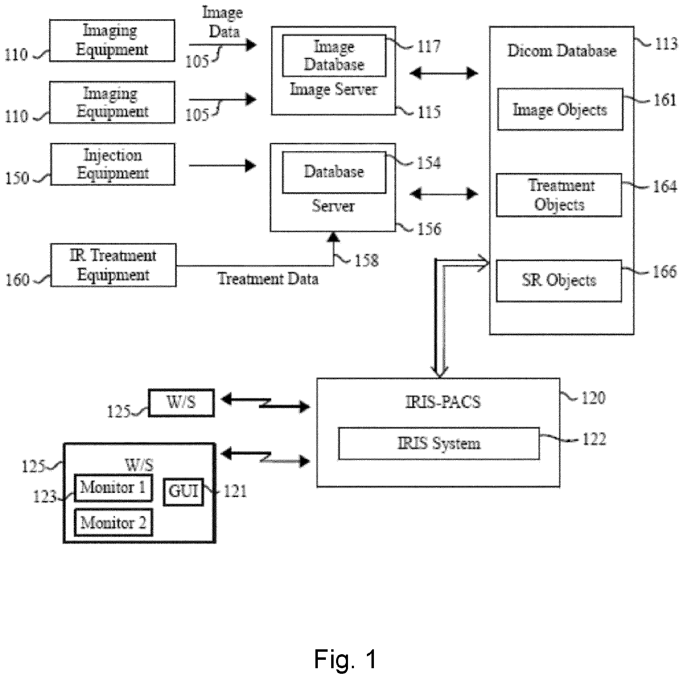

FIG. 1 illustrates a block diagram of an interventional radiology department. An interventional radiology department may include multiple rooms in which procedures are performed. The separate rooms may have dedicated systems and equipment installed therein. Additionally or alternatively, portable systems and equipment may be movable between procedure rooms. Various types of systems and equipment may be utilized during any single interventional radiology procedure. In general, radiology procedures involve one or more forms of imaging equipment/systems that afford the physicians the ability to view the region of interest during a procedure. Some types of imaging equipment utilize contrast agents in connection with imaging and as such one or more contrast agent injection systems may be included in the procedure room as well. Procedures also typically utilize various types of interventional equipment that may be used to provide access to the region of interest and/or two enable a treatment to be delivered.

By way of example only, FIG. 1 illustrates an environment that includes one or more interventional radiology imaging equipment 110, one or more injection systems 150 (e.g., contrast agents, etc.) 150, one or more interventional angiography systems 160, and one or more workstations 125. An interventional radiology information system (IRIS) may be provided as a stand-alone unit or integrated with a picture archive computing system (PACS), together collectively referred to as an IRIS-PACS 120. The IRIS-PACS 120 integrates data and images from numerous imaging equipment 110 modalities (x-ray, CT, NM, MRI, PET, SPECT, ultrasound, etc.). The IRIS-PACS 120 is configured to receive and process images from various types of imaging equipment's, therapy delivery systems, treatment planning systems, and the like. The IRIS-PACS is configured to receive images and other data from multiple healthcare providers, from different departments within a healthcare provider, etc. The IRIS functionality within the IRIS-PACS accesses, stores, updates and manages electronic patient records, and includes one or more modules for generating interventional radiology structured reports and advanced image visualization. The PACS functionality within the IRIS-PACS provides long-term archive and distribution of diagnostic images and patient related data.

Image data 105 is acquired by one or more imaging equipment 110 and stored in an image database 117 on an image server 115. Image data set 105 is then transferred to a DICOM database 113, using a communication protocol, such as one of the DICOM communication protocols. Optionally, the image data sets may be conveyed directly from the imaging equipment 110 to the DICOM database 113. The image data sets may represent two dimensional images and/or three-dimensional volumetric data sets.

Other data files stored in the DICOM database 113 may include information, referred to as non-image objects, relating to the way in which an image is to be presented. The non-image objects do not include image data but instead include other information related to the image data file(s) to which the non-image objects are to be applied. For example, the DICOM grayscale presentation state (GSPS) object stores the viewing parameters for a DICOM image objects 161 in order to allow for the consistent presentation of the images. A GSPS object may reference more than one image object 161 and, conversely, there may be more than one GSPS object which references a single image object. The DICOM database 113 also stores structured reporting objects 166, as well as other forms of objects and data defined in accordance with the DICOM standard. The data files stored in the DICOM database 113 are often saved into groups, called studies. Each study includes the data files which relate to one particular patient for a particular purpose. One study may include many different types of data files, including image data files such as DICOM Computed Tomography (CT) and Magnetic Resonance Imaging (MR) objects as well as non-image data files such as DICOM GSPS and SR objects.

It should be understood that the systems and modules may be implemented in hardware or software or a combination of both. With respect to the IRIS-PACS 120, modules of the interventional radiology information system 122 are preferably implemented in computer programs executing on programmable computers each comprising at least one processor, a data storage system, and at least one input and at least one output device. Without limitation, the programmable computers may be a server, personal computer, laptop, personal data assistant or cellular telephone. In some embodiments, the IRIS system 122 is installed on the hard drive of a user workstation 125, such that the user workstation 125 operates with the IRIS-PACS system 120 in a client-server configuration. The IRIS-PACS system 120 and the user workstation 125 both include client and server aspects relative to different functions. Optionally, the structure and function of the IRIS-PACS system 120 may be implemented on a stand-alone workstation. In other embodiments, the IRIS system 122 can run from a single dedicated workstation that may be associated directly with a particular imaging equipment 110. In yet other embodiments, the IRIS system 122 can be configured to run remotely on the user workstation 125 while communication with the PACS system occurs via a wide area network (WAN), such as through the internet.

The IRIS-PACS 120 is configured to access the image objects 161 stored in the DICOM database 113 in response to user commands through workstations 125, which may be a computer workstation consisting of a processor, display device and input devices (e.g., keyboard, mouse). The DICOM database 113 may represent a core component in many PACS systems. Alternatively, the workstation 125 may be a mobile device or any other suitable access device for displaying image data. Each image object 161 generated from the image data 105 can be expressed in logical parts. One part is known as pixel data that represents the displayed image. The other logical part is the metadata that represents a set of attributes that describes the image such as patient information, study grouping, and image attributes.

The IRIS-PACS 120 provides an interventional radiology structured reporting workflow, in which various aspects of the data entry for the structured report are presented through corresponding windows.

The IRIS system 122 may be provided integrated with a DICOM viewer in order to display the diagnostic images collected during the procedure, or to consult the previous exams. The workstation 125 includes a graphical user interface 121 and displays 123. The displays 123 may include 2 or more monitors. One monitor displays data sheets, worksheets, structured reports and other information as described and illustrated herein. The second monitor displays the corresponding images that are stored in the PACS system and provides a number of tools for visualization and image processing. The foregoing feature is provided by integration between the IRIS and the PACS, so that, by opening a specific procedure in the IRIS, the images - if present- are automatically loaded in the second monitor.

In addition, the IRIS system is configured to open previous images (acquired with the same modality or a different modality, such as XA/XA, or XA/CT, etc) of the same patient in order to perform a comparison of the clinical status.

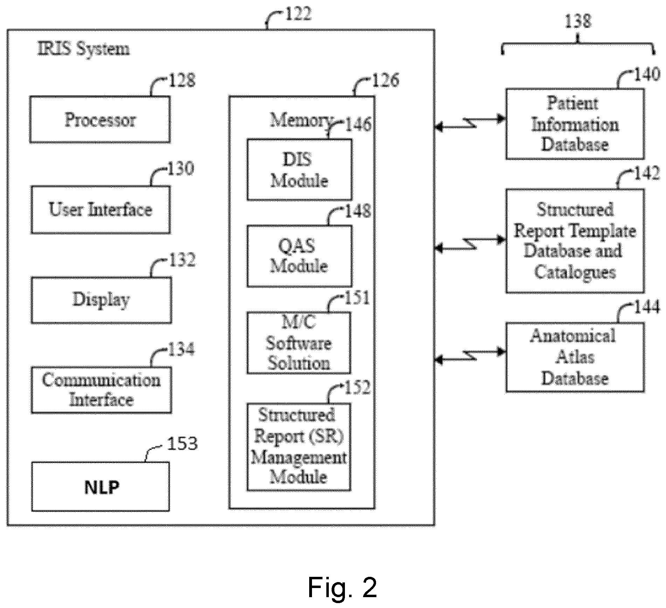

FIG. 2 illustrates an example block diagram of an interventional radiology information (IRIS) system 122 formed in accordance with embodiments herein. The IRIS system 122 includes one or more memory 126, one or more processors 128, a user interface 130, a display 132 and one or more communications interfaces 134. The memory 126 includes program instructions directing the one or more processors 128 to perform the operations described herein and implemented one or more software modules discussed herein in connection with managing workflow for structured reporting in connection with interventional radiology.

The IRIS system 122 communicates with one or more servers having memory 138 that store a patient information database 140, a structured report template database 142, and anatomical atlas database 144 and the like. Optionally, the structured report templates and anatomical atlases may be combined into one database. The patient information database 140 includes patient medical records and other information related to individual patients. The structured report template database 142 includes multiple structured report templates associated with corresponding interventional radiology procedures and classes of procedures. For example, each structured report template includes a set of data entry sheets, each of which has a predetermined format and data entry fields uniquely associated with particular aspects of a corresponding procedure and structured report. The anatomical atlas database 144 includes multiple anatomical atlases that correspond to unique portions of the vascular system. The anatomical atlases are not specific to any individual patient, but represent generic templates that may be developed based on models, multiple patients and the like. As one example, anatomical atlases may be developed, referred to as trait specific anatomical atlases, based upon a patient population that exhibits a particular characteristic or characteristics of interest, a disease of interest, demographic of interest, patient history, have undergone a particular past procedure and the like.

In the example of FIG. 2, the memory 138 of the servers are described as maintaining the various information as database structures. Optionally, the structured report templates, patient medical records and anatomical atlases may be stored in various formats and upon various medium that may include or not include database structures. The servers and/or memory 138 may be maintained at one or multiple locations, at one or multiple medical facilities, within one or more departments within a medical facility and/or upon one or more pieces of equipment. By way of example, the patient information database 140 may be distributed across multiple physical locations within a medical network or across multiple medical networks. The structured report template database 142 and anatomical atlas database 144 may be maintained upon a single device/workstation or distributed across multiple locations. As one example, a base set of structured report templates and a base set of anatomical atlases may be maintained within memory 126 on the IRIS system 122, while additional structured report templates and anatomical atlases may be maintained elsewhere, within or outside of a medical facility or medical network.

The memory 126 includes, among other things, a diagnostic imaging software module 146 that is configured to provide two-dimensional, three dimensional and four-dimensional image processing of two-dimensional and volumetric imaging data sets from various modalities (e.g., CT, x-ray, pet, SPECT, ultrasound, MRI, etc.). The memory 126 includes a quantitative analysis software module 148 that is configured to perform quantitative analysis upon diagnostic images, and other patient data. The memory 126 further includes a mobile/cloud software solution 151 that enables clinical collaboration and results distribution over a wide geographic region and to a variety of end computing devices.

The memory 126 also includes a Structured Report (SR) management module 152 that interacts with the Structured Report Template Database and Catalogues 142 to load the appropriate template depending on type of procedure and analysis. The Structured Report (SR) Management Module 152 includes, or is in communication with, an NLP module 153 devoted to the conceptual and logical mapping of free text reports into the loaded structured template as it will be explained in the next sections. The free text reports may be written or dictated by the clinician through the user interface 130 or retrieved from the servers and/or memory 138, for example from the Patient Information Database 140, or other database connected or connectible through the Communication Interface 134.

The processor 128 provides vendor neutral enterprise archives of patient records and structured reporting results to enable broad compatibility and interoperability of products and technologies, both in connection with DICOM and non-DICOM images.

The communications interface 134 affords access to external resources, such as medical networks, interventional radiology equipment and the like.

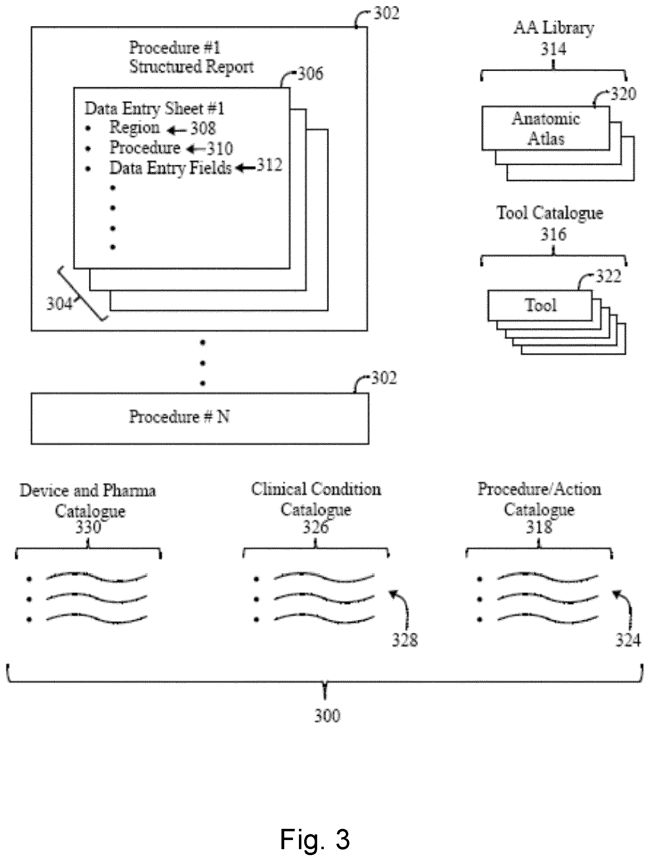

FIG. 3 illustrates an example of at least a portion of the resources utilized in an interventional radiology structured (IRS) workflow 300. The IRS workflow 300 includes multiple structured reports 302, each of which is specific to a particular interventional procedure. Each structured report 302 includes a set 304 of data entry sheets 306. Each data entry sheet includes a predetermined format and data entry fields uniquely associated with a particular aspect or aspects of the procedure and structured report. At least one of the data entry sheets 306 includes an anatomical region field 308, a procedure designation field 310, and various other data entry fields 312. The anatomical region field 308 designates the portion of the patient's vascular system, for which the interventional procedure is carried out. The procedure designation field 310 designates the type or nature of the procedure carried out.

Prior art systems require the physician to fill the data entry sheets of the structured report template which has been selected for the specific procedure.

The idea at the basis of the present disclosure is to automatically translate free text medical reports in fields of corresponding data entry sheets of a structured report workflow.

The problem of translating a natural language medical report into a structured report is a subcategory of the problems of structured prediction, widely diffused within computational linguistics. The approaches of structured prediction are usually characterized by two steps: one of information extraction, in which an input document is processed in order to extract the relevant information, and one of inference, in which the information obtained is used to predict the value associated with one or more fields with numerical or categorical values.

Natural language processing (NLP) is the ability for computers to understand the latest human speech terms and text.

The adoption of natural language processing in healthcare is rising because of its recognized potential to search, analyze and interpret large amounts of patient datasets. Using advanced medical algorithms, machine learning in healthcare and NLP technology services have the potential to harness relevant insights and concepts from data that was previously considered buried in text form. NLP in healthcare media can accurately give voice to the unstructured data of the healthcare universe, giving incredible insight into understanding quality, improving methods, and better results for patients.

Physicians spend a lot of time inputting the clinical reports of their patients into text notes. These notes aren't easily extractable in ways the data can be analyzed by a computer and get stored and sent to EHR as free text.

Huge volumes of unstructured patient data are inputted into EHRs on a daily basis, but it's hard for a computer to help physicians aggregate that critical data. Big data analytics in healthcare shows that up to 80 percent of healthcare documentation is unstructured, and therefore goes largely unutilized, since mining and extraction of this data is challenging and resource intensive. Without extracting structured information, that data is not in a usable format for modern computer-based algorithms to compare and retrieve relevant information. NLP techniques can help to pre-process the data in order to structure information from free text and help the following process of classification and categorization. In addition, NLP output can be used as an input for systems devoted to check previously missed or improperly coded patient conditions.

A distinct advantage natural language processing medical records offers is the ability for computer assisted coding to synthesize the content of long text notes into just the important points. Historically, this could take organizations weeks, months, even years, to manually review and process stacks of chart notes from health records, just to identify the pertinent info. Natural language processing software for healthcare can scan clinical text within seconds and identify what needs to be extracted. This frees up physicians and staff resources to focus more on the complex matters and reduces the time spent on redundant administrative policy. When computers can understand physician notation accurately and process that data accordingly, valuable decision support can be obtained.

Another advantage of NLP tools is that its accuracy goes up along with the volume of data available for learning. The more a medical NLP platform is used, the more accurate using artificial intelligence in healthcare gets, since it's always learning, and in some cases, can be customizable.

The data extraction from a natural language text can be approached as a problem of text classification. In practice, given a piece of text (called "context"), the goal is determining if a given feature (e.g., the presence of fever) is present or not, or if the information about that is absent from the context (not mentioned at all). A traditional approach would have required many manually classified examples to achieve acceptable performance. It would have been necessary to train the specific classification models for each information that should be extracted from the text, using an LSTM architecture (Sepp Hochreiter and Jürgen Schmidhuber. Long short-term memory. Neural computation, 9(8):1735-1780, 1997) or GRU (Gated Recurrent Unit).

More recently, innovative systems for pre-training based natural language models development have been introduced (BERT: Pre-training of Deep Bidirectional Transformers for Language Understanding - arXiv:1810.04805v2 [cs.CL] 24 May 2019), together with more efficient architectures based on text comprehension (Attention Is All You Need - arXiv:1706.03762 [cs.CL] 6 Dec 2017). This pre-training step is particularly powerful because it allows the model to learn using a dataset not previously manually classified or tagged.

In particular, using a self-supervision approach in which the model is only asked to "guess" a given word removed from a certain context, the model can learn the semantic relationships between words. Given the large availability of free text on the internet, there is no difficulty in finding gigabytes or even terabytes of text on which the model can be pre-trained to understand such semantic relationships. Some complex models may even perform information extraction tasks without performing any training step (zero-shot learning) or with very few classified data (few-shot learning) (Language Models are Few-Shot Learners-arXiv:2005.14165v4 [cs.CL] 22 Jul 2020).

Due to the difficulty and the costs of finding pre-classified data to use for training steps, the self-supervision approaches are now the state-of-the-art for NLP tasks.

For the implementation of the present system a pre-trained model with T5 (Text-to-Text Transfer Transformer - arXiv:1910.10683) architecture has been chosen. It has been proven by scientific literature that it is effective for several kinds of tasks although it can run on a light workstation, like a desktop PC.

In order to fully exploit the capabilities of modern natural language processing systems without compromising the effectiveness and generalizability of the approach, a Question Answering (QA) (Khashabi et al. 2020, "UNIFIEDQA: Crossing Format Boundaries with a Single QA System", Findings of the Association for Computational Linguistics: EMNLP 2020 - arXiv:2005.00700v3 [cs.CL] 7 Oct 2020) model has been adopted, as it gives the capability to work in zero shot or few shots mode and allows the user to easily configure the model for the extraction of a new field.

The chosen model, called UnifiedQA, is a single pre-trained QA system that exploits information across 4 different QA formats to achieve strong performance across 20 different factoid and commonsense QA datasets, so building a format-agnostic QA system, based on neural networks and pre-trained in various formats (extractive, abstract, multiple choice and true / false).

It is parameterized to answer questions in a sequence-to-sequence format (Sutskever, Ilya, Oriol Vinyals and Quoc V. Le. "Sequence to Sequence Learning with Neural Networks." NIPS 2014), given a context to extract the information from and a list of available choices. The training scripts and trained weights are open sourced with commercial use licenses. The intuition behind the choice of this model is to cast multiple QA problems (Yes/No, Multiple choice, extractive and abstractive) into a text-to-text paradigm to improve generalization capabilities, making it perfect for the specific use case.

The generalizability of this model makes it applicable to various types of medical reports, without requiring an ad-hoc training procedure for each and has been chosen as a first step of the proposed system. The system, in fact, aims to be a general system for structuring report, without requiring lots of pre-classified texts and huge quantities of medical reports for training the classifier. One of the main features of the proposed system is the capability to use the same architecture to train the neural network to recognize the desired entities and then, once the structured report is ready, use the structured information to adapt and optimize the other reporting tools, for instance image visualization, clinical history retrieval, similar cases comparison, consistency and coherence report check.

For such reasons, the system has been initially trained and tested on radiological reports of CT thorax examinations of suspected COVID-19 infections but can be easily extended to other disciplines and examinations, not only in traditional and interventional radiology but even outside the radiology field.

At the moment, the UnifiedQA system is available only in English language, so the first step of the process requires the translation of the text into English, automatically performed by using a neural machine translation model (MTM). This step has to be considered optional and temporary depending on the operating language.

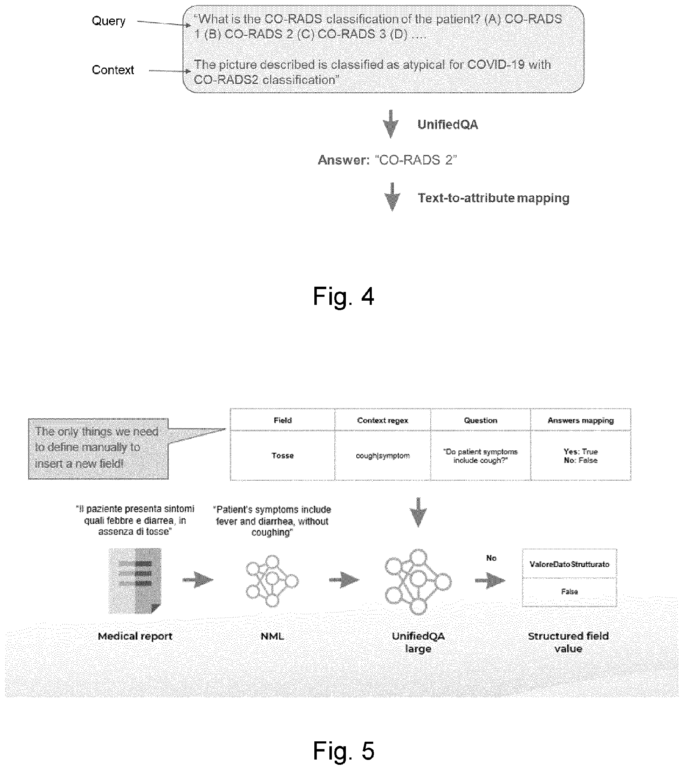

Once the translation has been made and the context has been identified, the UnifiedQA system answers a predefined question associated with the category as exemplary shown in FIG. 4

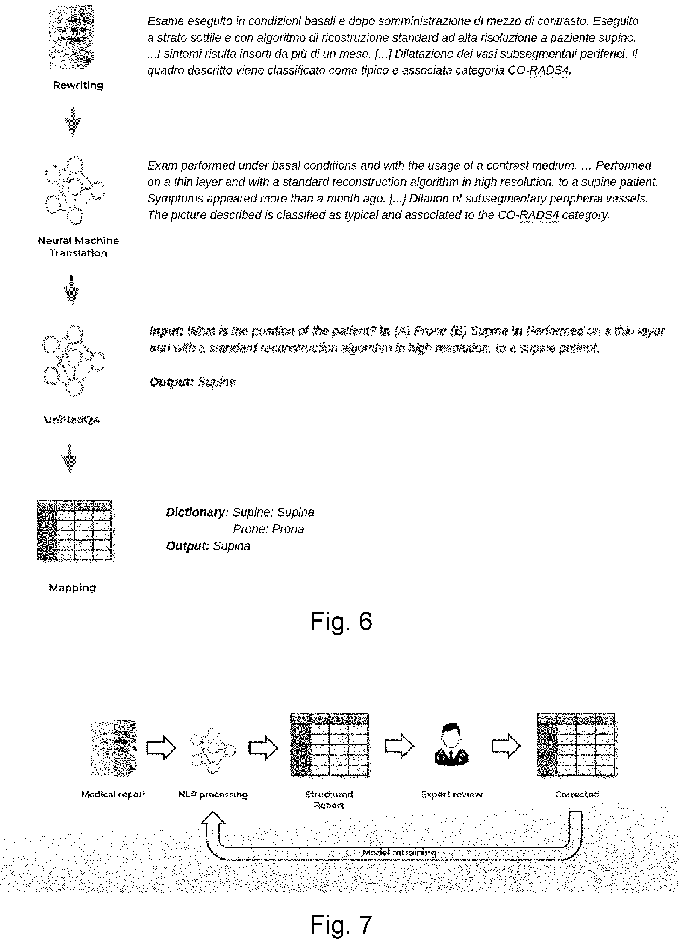

The output is finally mapped to a dictionary of possible values for the category itself. The architecture of the solution is summarized in figures 5 and 6.

Optionally, a continuous learning architecture, as shown in FIG. 7, can be inserted in the reporting system, in order to allow the model retraining and an increase of performance in terms of inference percentage.

In its more general structure, a system for managing a medical structured reporting workflow according to embodiments herein comprises:
a workstation (125) including a graphical user interface (121) and a display (123);
memory (126) to store program instructions and a medical structured workflow including multiple structured reports (142), the structured reports associated with corresponding medical procedures, the structured reports including sets of data entry sheets having a predetermined format and data entry fields uniquely associated with a corresponding aspect of a procedure;
a processor (128) configured to execute the program instructions to:
   receive (801) a structured reporting workflow designator designating an individual structured report template from a class of structured report templates, such structured report templates having fields to be populated with data received in reply to a specific question, for example by selecting from a list of predefined items, wherein each item of each list is associated to a specific answer to a specific question;
   receive (802) a free text -written or dictated- medical report related to the designated structured report template;
   process (803) the free text medical report with a machine learning algorithm to determine answers to the questions associated to the fields of the designated structured report template;
   use (804) the answers to populate the fields of the designated structured report template with associated data.

This represents only the first step towards an intelligent system for the management of unstructured clinical information. The structured information that is extracted from the text report, based on pre-configured templates, can, in fact, be used as input to different classification algorithms that can improve the reliability and the performances of an Enterprise Imaging Reporting System, helping the referring physician to speed up and qualitatively improve the reading and the interpretation of the medical images and the patient clinical data.

The system may be used, for example, to manage a medical structured reporting workflow including examination images of a patient in conjunction with a PACS, either connected, connectible or included in the system, In this case, the processor may be advantageously configured to populate the fields of a report template from a free text imaging examination report and use such populated fields to automatically load from the PACS previous examination studies of the patient in the form of raw or processed images optionally applying optimized visualization protocols to facilitate comparison between the images.

Further examples of algorithms and applications are described in the following sections.

### FIELDS OF APPLICATION

### Immediate access to the summary of the clinical history ("Accelerating EMR chart review")

In order to optimize the workflow and reduce reporting times, a use case linked to the use of automatic structured reporting may consist in providing medical staff with a dashboard in which the salient events that distinguish the clinical history are summarized.

After using NLP techniques to extract structured information from each report present in the clinical database, a "weight" is associated with each type of information extracted (template entity) while a tag identifies the category (e.g. clinical question, familiar history, previous pathologies, ongoing therapies, diagnosis, etc.). Each category has its own weight and within the category each entity has another weight. Optionally, for some selected categories it is possible to dynamically change the weight of the entities taking into account a temporal criterion (in which more recent information is attributed a greater weight)

To draw up the clinical summary, a weighted combination of the various entities is then used and only those whose value exceeds a predefined threshold are taken into consideration.

Entities that are repeated within multiple reports can be given greater importance and be highlighted on a graphic level (text in bold, or larger font).

By clicking on the text of the entities, the system can open the list of the reports that contributed to generate the score and also their associated images.

Through such tools, it is possible for the referring physician to have an immediate picture of the patient's clinical status at the time of the current examination and use this data to get a more complete diagnosis.

Optionally, in the case of integration with PACS viewer, the identification of the most relevant exams starting from the content of the report, combined with the intelligent analysis of the diagnostic question of the current exam, also may allow to automatically load the most relevant previous imaging studies, apply optimized visualization protocols to facilitate comparison with the current exam and pre-process images through traditional post-processing tools and/or AI algorithms.

### Example of use case:

The patient arrives in the Emergency Department presenting chest pain and breathing difficulties and, after some preliminary examinations, he is prescribed a chest CT scan for suspected pulmonary embolism.

A software tool, on the basis of the type of investigation prescribed and the clinical question, verifies, starting from the previous reports of that patient archived in the clinical database, whether current pulmonary embolism or pulmonary nodules have been observed in previous examinations (for example looking for the word "embolism" or "pulmonary nodules" in the fields of the structured report as extracted), giving evidence (facilitating access to the corresponding reports via a link) and automatically extracting all relevant data from the clinical report history including anamnestic data, therapies in progress, previous pathologies.

### Extraction of significant features for patient classification and follow-up

In order to help the doctor to understand the patient conditions, AI techniques and more specifically NLP techniques allow to classify the anamnestic and/or therapeutic data in order to create a scientific database and then compare these data with similar cases and / or guidelines to provide additional information that can help the physician to predict the outcome of the procedure and its follow up over time.

In this case, first the report is dictated or written, then NLP techniques are applied to extract the relevant information based on the predefined template.

Finally, the set of structured information thus extracted is compared with the sets of information present in the clinical reference database. The comparison takes place by attributing to each entity a weight and using an algorithm to calculate the global similarity score.

For each report that obtains a score higher than a predefined threshold, the most relevant information is shown in order to provide indications for the continuation of the treatment (diagnosis, therapies, follow up).

### Clinical risk reduction

In order to reduce the risks associated with incorrect reports, another use related to the use of AI techniques consists in providing medical staff with a tool capable of checking inconsistency and providing alerts about:
- gender;
- laterality / part of the body;
- previous examinations

In this scenario, NLP techniques are used to extract entities. Semantic validation rules are then applied to some of these entities, pre-configured in the system (e.g. organ / gender association, examination laterality / organ laterality indicated in the report, presence or absence of the contrast medium in the examination technique with respect to the examination performed, consistency between measurements and diagnosis, etc.), as well as checks relating to the mandatory presence of certain information.

Optionally, consistency checks can also be performed by matching the data extracted from the reporting system using NLP techniques with the DICOM tags associated with the imaging studies and stored in the PACS system.

### Example: Identification of gender error

a. The radiologist looks at an abdomen CT scan of a female patient
b. During the dictation / writing of the report, information about the prostate and seminal vesicles are inserted
c. The system automatically shows the identified inconsistency with an alert (e.g. red text).

### Report Classification and Content-Based Image Retrieval

One of the physicians' recurring requests is providing a system that allows for easy-to-use classification of studies. One of the major limitations is the impossibility of carrying out a semantic search within the PACS VNA archive using specific clinical content.

To meet this need, using AI techniques, the system, upon closing the report and by means of NLP techniques, automatically may extract entities, made available in the form of TAGs, which the doctor could confirm and / or modify to allow both the classification for scientific purposes (teaching file) and to improve the training of the NLP system.

These tags may be identified starting from the entities extracted in the template, applying a weight of relevance to them (for example, the diagnoses represent a more important tag than the clinical question or other symptomatology). The system could propose a set of them and the doctor could then validate the choice. In some cases, it could be possible using semantic rules for combining multiple entities into a single derived tag.

The system, depending on what is classified, could then allow both to carry out one
- search by TAG
- semantic search

### Attribution Level of priority in reporting - Workflow optimization

Another possible scenario for the use of AI techniques is related to the identification of criteria that can change the priority level of an examination within the reporting list. This scenario finds an easier implementation when it is possible to have imaging analysis tools (able to identify abnormal situations). This activity is usually carried out implicitly by the radiologist who identifies which organs or parts of organs have anomalies compared to a normal condition. A support for the attribution of priority can be obtained using NLP techniques applied to the textual part (in particular to the diagnostic question and to the medical history data present in the EPR/PACS VNA system) in order to identify critical conditions and then assign an appropriate level of priority.

Also in this case a weight may be attributed to the relevant entities.
The score that determines the priority of an exam derives from a weighted combination of the chosen entities and the scores deriving from the image processing tools.

The embodiments described herein may include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network ("SAN") familiar to those skilled in the art. Similarly, any necessary files for performing the functions attributed to the computers, servers or other network devices may be stored locally and/or remotely, as appropriate. Where a system includes computerized devices, each device can include hardware elements that may be electrically coupled via a bus, the elements including, for example, at least one central processing unit ("CPU" or "processor"), at least one input device (e.g., a mouse, keyboard, controller, touch screen or keypad) and at least one output device (e.g., a display device, printer or speaker). Such a system may also include one or more storage devices, such as disk drives, optical storage devices and solid-state storage devices such as random access memory ("RAM") or read-only memory ("ROM"), as well as removable media devices, memory cards, flash cards, etc.

Such devices also can include a computer-readable storage media reader, a communications device (e.g., a modem, a network card (wireless or wired), an infrared communication device, etc.) and working memory as described above. The computer-readable storage media reader can be connected with, or configured to receive, a computer-readable storage medium, representing remote, local, fixed and/or removable storage devices as well as storage media for temporarily and/or more permanently containing, storing, transmitting and retrieving computer-readable information. The system and various devices also typically will include a number of software applications, modules, services or other elements located within at least one working memory device, including an operating system and application programs, such as a client application or web browser. It should be appreciated that alternate embodiments may have numerous variations from that described above. For example, customized hardware might also be used and/or particular elements might be implemented in hardware, software (including portable software, such as applets) or both. Further, connection to other computing devices such as network input/output devices may be employed.

Various embodiments may further include receiving, sending, or storing instructions and/or data implemented in accordance with the foregoing description upon a computer-readable medium. Storage media and computer readable media for containing code, or portions of code, can include any appropriate media known or used in the art, including storage media and communication media, such as, but not limited to, volatile and non-volatile, removable and non-removable media implemented in any method or technology for storage and/or transmission of information such as computer readable instructions, data structures, program modules or other data, including RAM, ROM, Electrically Erasable Programmable Read-Only Memory ("EEPROM"), flash memory or other memory technology, Compact Disc Read-Only Memory ("CD-ROM"), digital versatile disk (DVD) or other optical storage, magnetic cassettes, magnetic tape, magnetic disk storage or other magnetic storage devices or any other medium which can be used to store the desired information and which can be accessed by the system device. Based on the disclosure and teachings provided herein, a person of ordinary skill in the art will appreciate other ways and/or methods to implement the various embodiments.

The specification and drawings are, accordingly, to be regarded in an illustrative rather than a restrictive sense. It will, however, be evident that various modifications and changes may be made thereunto without departing from the broader scope of the invention as set forth in the claims.

Thus, while the disclosed techniques are susceptible to various modifications and alternative constructions, certain illustrated embodiments thereof are shown in the drawings and have been described above in detail. It should be understood, however, that there is no intention to limit the invention to the specific form or forms disclosed, but on the contrary, the intention is to cover all modifications, alternative constructions and equivalents falling within the scope of the invention, as defined in the appended claims.

The use of the terms "a" and "an" and "the" and similar referents in the context of describing the disclosed embodiments (especially in the context of the following claims) are to be construed to cover both the singular and the plural, unless otherwise indicated herein or clearly contradicted by context. The terms "comprising," "having," "including" and "containing" are to be construed as openended terms (i.e., meaning "including, but not limited to,") unless otherwise noted. The term "connected," when unmodified and referring to physical connections, is to be construed as partly or wholly contained within, attached to or joined together, even if there is something intervening. Recitation of ranges of values herein are merely intended to serve as a shorthand method of referring individually to each separate value falling within the range, unless otherwise indicated herein and each separate value is incorporated into the specification as if it were individually recited herein. The use of the term "set" (e.g., "a set of items") or "subset" unless otherwise noted or contradicted by context, is to be construed as a nonempty collection comprising one or more members. Further, unless otherwise noted or contradicted by context, the term "subset" of a corresponding set does not necessarily denote a proper subset of the corresponding set, but the subset and the corresponding set may be equal.

Operations of processes described herein can be performed in any suitable order unless otherwise indicated herein or otherwise clearly contradicted by context. Processes described herein (or variations and/or combinations thereof) may be performed under the control of one or more computer systems configured with executable instructions and may be implemented as code (e.g., executable instructions, one or more computer programs or one or more applications) executing collectively on one or more processors, by hardware or combinations thereof. The code may be stored on a computer-readable storage medium, for example, in the form of a computer program comprising a plurality of instructions executable by one or more processors. The computer-readable storage medium may be non-transitory.

Preferred embodiments of this disclosure are described herein, including the best mode known to the inventors for carrying out the invention. Variations of those preferred embodiments may become apparent to those of ordinary skill in the art upon reading the foregoing description. The inventors expect skilled artisans to employ such variations as appropriate and the inventors intend for embodiments of the present disclosure to be practiced otherwise than as specifically described herein. Accordingly, the scope of the present disclosure includes all modifications and equivalents of the subject matter recited in the claims appended hereto as permitted by applicable law. Moreover, any combination of the above-described elements in all possible variations thereof is encompassed by the scope of the present disclosure unless otherwise indicated herein or otherwise clearly contradicted by context.

## Claims

1. A computer-implemented method for managing a medical structured reporting workflow, the method comprising:
a) receiving a structured reporting workflow designator designating an individual structured report template from a class of structured report templates, such structured report templates having fields to be populated with data received in reply to a specific question;
b) receiving a free text medical report related to the designated structured report template;
c) processing the free text medical report with a machine learning algorithm to determine answers to the questions associated to the fields of the designated structured report template;
d) using the answers to populate the fields of the designated structured report template with associated data.

2. Method according to claim 1, wherein such structured report templates have fields to be populated by selecting from a list of predefined items, wherein each item of each list is associated to a specific answer to a specific question.

3. Method according to claim 1 or 2, wherein the machine learning algorithm is of the question-answering type pre-trained on known datasets of questions-answers including one or more of the following formats: Extractive where questions include a context paragraph, typically a paragraph, and require models to extract the answer as a substring from the context, Abstractive where questions require models to produce answers that are often not mere substrings of the provided context paragraph, Multiple-choice where questions have a set of candidate answers of which generally exactly one is correct, Yes/No where questions expect a 'yes' or 'no' answer as the response.

4. Method according to any preceding claim, wherein the machine learning algorithm is the UnifiedQA algorithm.

5. Method according to any preceding claim, wherein the free text medical report is translated before being processed.

6. Method according to any preceding claim, wherein the populated fields of the designated structured report template is subjected to a review for being corrected, the fields as corrected being used for retraining the machine learning algorithm.

7. Method according to any preceding claim, wherein all or some of the populated fields of the structured report template are used as input to classification algorithms to improve the reliability and the performances of an Enterprise Imaging Reporting System.

8. Method according to any preceding claim, wherein all or some of the fields of the structured report templates are associated with entities having different weights (We), the method further comprising building a clinical summary of a patient from free text medical reports related to the patient obtained at different time by providing an ordered sequence of entities starting from the one having the higher weight, ignoring those entities having weights below a pre-determined threshold.

9. Method according to claim 8, wherein the entities are grouped in categories identified by tags having different weights (Wc), the weight of the categories being used to modify the weight of the associated entities to obtain a modified weight to be used to determine the ordered sequence of entities.

10. Method according to claim 8 or 9, wherein further weights are used to determine the ordered sequence of entities including a temporal weight providing a higher relevance to more recent information and/or a repetition weight providing a higher relevance to recurrent entities.

11. Method according to any preceding claim, wherein the extracted structured information of a patient is compared with sets of information present in a clinical reference database by attributing to each field a weight and using an algorithm to calculate a global similarity score to identify past exams of the same patient or similar exams of other patients and provide the most relevant information for patient diagnosis and/or follow-up.

12. Method according to any preceding claim, wherein the extracted structured information of a patient is checked for inconsistency through semantic validation rules to provide alerts.

13. Method according to claim 12, wherein an inconsistency is reported if one or more of the following situation occurs: an organ is not compatible with the gender, the examination/organ laterality is not compatible with the laterality extracted from the report, presence or absence of the contrast medium in the examination technique with respect to the examination performed, consistency between measurements and diagnosis, mandatory information not present, data extracted not matching DICOM tags associated with the imaging modality and stored in a PACS system.

14. Method according to any preceding claim, wherein all or part of the extracted entities populating the field of the structured report template are made available in the form of TAGs that can be confirmed or modified by a user to allow the classification for scientific purposes and/or to improve the training of the machine learning algorithm.

15. Method according to claim 14, further comprising applying a weight of relevance to the TAGs before receiving a validation input from the user, possibly using semantic rules for combining multiple fields or entities into a single derived tag.

16. Method according to any preceding claim, further comprising assigning a priority level to an examination by identifying in the associated free text medical report critical conditions related to a diagnostic question.

17. A computer product directly loadable into the memory of a digital computer and comprising software code portions for performing the method according to any of the preceding claims when the product is run on a computer.

18. A computer system for managing a medical structured reporting workflow, the system comprising:
a workstation (125) including a graphical user interface (121) and a display (123);
memory (126) to store program instructions and a medical structured workflow including multiple structured reports (142), the structured reports associated with corresponding medical procedures, the structured reports including sets of data entry sheets having a predetermined format and data entry fields uniquely associated with a corresponding aspect of a procedure;
a processor (128) configured to execute the program instructions to:
receive (801) a structured reporting workflow designator designating an individual structured report template from a class of structured report templates, such structured report templates having fields to be populated with data received in reply to a specific question;
receive (802) a free text medical report related to the designated structured report template;
process (803) the free text medical report with a machine learning algorithm to determine answers to the questions associated to the fields of the designated structured report template;
use (804) the answers to populate the fields of the designated structured report template with associated data.

19. Computer system according to claim 18 for managing a medical structured reporting workflow including examination images of a patient, wherein the processor (128) is configured to populate the fields of a report template from a free text imaging examination report and use such populated fields to automatically load from a PACS, either connected, connectible or included in the system, previous examination studies of the patient in the form of raw or processed images optionally applying optimized visualization protocols to facilitate comparison between the images.
